# FASCICULE DE BREVET EUROPEEN

(11) **EP 1 560 953 B1**
(45) Date de publication et mention de la délivrance du brevet: **24.03.2010**
(21) Numéro de dépôt: 03767910.7
(22) Date de dépôt: 05.11.2003
(51) Int. Cl.: D01F 1/10, D01F 6/60

(54) **ARTICLES A ACTIVITE ANTIBACTERIENNE ET ANTIFONGIQUE**
ARTIKEL MIT ANTIBAKTERIELLER UND FUNGIZIDER AKTIVITÄT
ARTICLES WITH ANTIBACTERIAL AND ANTIFUNGAL ACTIVITY

(30) Priorité: 08.11.2002 FR 0214007
(43) Date de publication de la demande: 10.08.2005
(73) Titulaire: Rhodianyl, 92512 Boulogne-Billancourt Cédex (FR)
(72) Inventeur: CHARBONNEAUX, Thierry, 69006 Lyon (FR); ROCHAT, Sandrine, F-69100 Villeurbanne (FR)
(74) Mandataire: Esson, Jean-Pierre
(86) Numéro de dépôt international: PCT/FR2003/003298
(87) Numéro de publication internationale: WO 2004/044284

(56) Documents cités:
- EP-A- 0 557 880
- WO-A-94/15462
- WO-A-99/67451
- WO-A-03/056923

## Description

La présente invention concerne des articles ayant une activité antibactérienne et antifongique comprenant du sulfure de zinc. Les fils, fibres, filaments et articles selon la présente invention peuvent notamment être mis en oeuvre dans la fabrication de tout produit susceptible d'être mis en contact avec des bactéries et/ou champignons, tels que par exemple des vêtements, tapis, rideaux, literies et les matériaux textiles médicaux. La présente invention concerne l'utilisation de sulfure de zinc pour la fabrication de fils, fibres, filaments et/ou articles à propriétés antibactérienne et antifongique.

Dans de nombreuses applications telles que le domaine textile, on cherche à limiter le développement des bactéries et des champignons, dans un but de prévention des affections chez l'homme et pour éviter les mauvaises odeurs. Dans les secteurs médicaux, par exemple, il est également de grande importance de limiter le développement des bactéries et des champignons sur les outils de travail, sur les matériaux de construction et sur les vêtements.

De nombreux agents présentant des propriétés biocides sont connus depuis fort longtemps et sont utilisés dans différentes applications. Parmi ces agents, les éléments à base de métaux tels que l'argent, le cuivre ou le zinc, d'ammonium quaternaire, ou à base organique comme le triclosan sont les plus connus.

Afin de conférer aux surfaces textiles des propriétés biocides, de nombreux apprêts contenant des composés bioactifs ont été développés. Toutefois ces apprêts ont toujours une tenue limitée et leur effets disparaissent après un ou plusieurs lavages. Il est donc dans de nombreux cas plus intéressant d'introduire le principe actif directement dans l'article devant présenter une propriété bioactive.

De nombreux agents antibactériens et antifongiques commerciaux sont connus. Ces agents ne peuvent toutefois pas être introduits dans des matrices polymériques, puisqu'ils ne résistent pas aux températures de mises en forme de ces derniers, et peuvent être transformés à ces températures ou interagir avec la matrice.

On recherche toujours de nouveaux agents antibactériens et antifongiques de faible coût et aisées à mettre en oeuvre dans des articles à base de matrice polymérique.

La demanderesse a mis en évidence que des fils, fibres, filaments et/ou articles, tels que des films, comprenant du sulfure de zinc (ZnS) dans leur matrice polymérique, possèdent d'excellentes propriétés antibactérienne et antifongique.

Ces propriétés antimicrobiennes sont conférées par l'ajout de sulfure de zinc comme charge minérale dans la matrice polymérique.

Le sulfure de zinc se disperse aisément dans la matrice polymérique ce qui permet une répartition uniforme de ce composé dans les fils, fibres, filaments et/ou articles. Le sulfure de zinc ne s'agglomère pas dans la matrice polymérique contrairement à de nombreuses particules à base de métaux connus de l'art antérieur comme agent antimicrobien.

Par diffusion et migration, le principe actif, sous forme de sulfure de zinc et/ou de zinc, est relargué à la surface des fils, fibres, filaments et/ou articles et entre en contact avec l'environnement comprenant les bactéries et les champignons, ce qui permet une plus longue activité antibactérienne et antifongique dans le temps. Lors du lavage des fils, fibres et/ou filaments, il se produit une légère élimination du principe actif en surface. Toutefois, la diffusion du principe actif dans la matrice polymérique du coeur vers la surface des fils, fibres, filaments et/ou articles permet le maintient constant de l'activité antibactérienne et antifongique. Cette activité est ainsi préservée très longtemps en dépit des lavages des fils, fibres, filaments et/ou articles.

Le sulfure de zinc présente également l'avantage de résister aux températures de mise en forme de la matrice thermoplastique. Le sulfure de zinc n'est donc pas modifié ou altéré à ces températures.

De plus, le sulfure de zinc est inerte et ne réagit pas avec la matrice polymérique ce qui ne cause pas de problème de dégradation, de coloration, de jaunissement des fils, fibres, filaments et/ou articles, contrairement aux agents antimicrobiens de l'art antérieur, comme l'oxyde de zinc (ZnO) ou l'argent (Ag) par exemple. De plus, les fils, fibres, filaments et/ou articles comprenant du sulfure de zinc ne sont pas abrasifs.

Le sulfure de zinc permet également de satisfaire les propriétés recherchées au niveau du coût, de la facilité de mise en oeuvre et de l'introduction dans des matrices polymériques, tels que les matrices thermoplastiques. Le sulfure de zinc présente également l'avantage d'être un bon agent matifiant.

On entend par antibactérien, l'action visant à limiter, réduire ou éliminer les bactéries présentes dans un environnement. Par bactérie on entend les eubactéries et les archéobactéries. Les eubactéries incluent les fermicutes, les gracilicutes et les ternicutes. Les gracilicutes incluent les bactéries Gram négatives telles que les Enterobacteriaceae, comme par exemple Klebsiella (telle que *Klebsiella pneumoniae*) et Escherichia (telle que *Escherichia coli*). Les fermicutes incluent les bactéries Gram positif, telles Micrococcaceae, comme par exemple les Staphylocoques (tel que *Staphylococcus aureus*) et les tiges formant des endospores incluant les bacilles (Bacillaceae) comme par exemple *Bacillus circulans*. Toutes ces références sont mentionnées dans le Bergey's Manual of Systematic Bacteriology, Williams & Wilkens, 1st ed. Vol. 1-4, (1984).

On entend par antifongique, l'action visant à limiter, réduire ou éliminer les champignons (mycètes) présentes dans un environnement. Le terme Myceteae inclus Amastigomycota comme par exemple Deuteromycotina qui inclus les Deuteromycetes. Les Deuteromycetes incluent Aspergillis (*Aspergillus niger*) et Candida (*Candida albicans*).

Par environnement, on entend tout milieu comprenant au moins des bactéries et/ou des champignons. L'environnement peut être un liquide ou un gaz, de préférence l'air. Par réduire, on entend diminuer la quantité de bactéries et/ou champignons présents dans l'environnement, comparée à la quantité présente dans l'environnement avant l'introduction de fils comprenant du sulfure de zinc. Par réduire, on entend également réduire le taux de croissance des nouvelles bactéries et/ou champignons dans le temps et dans l'environnement. Par réduire, on entend aussi réduire le taux de reproduction des bactéries et/ou champignons. Par éliminer, on entend éliminer de l'environnement la majorité des bactéries et/ou champignons, c'est-à-dire tuer les bactéries et/ou champignons présents dans l'environnement ou les rendre inactifs. Par éliminer, on entend également prévenir la croissance de nouvelles bactéries et/ou champignons.

La présente invention concerne l'utilisation de sulfure de zinc dans une matrice polymérique pour la fabrication de fils, fibres, filaments et/ou articles à propriétés antibactérienne et antifongique. Le sulfure de zinc y joue le rôle d'agent antibactérien et antifongique.

La présente invention comme objet l'utilisation de sulfure de zinc dans une matrice polymérique pour de fabrication de fils, fibres, filaments ou articles à propriétés antibactérienne et antifongique.

La présence de sulfure de zinc dans une matrice polymérique peut être déterminée par différentes méthodes bien connues de l'homme du métier, telles qu'une analyse directe qualitative des éléments zinc et soufre par spectrométrie de fluorescence X ; éventuellement suivi d'un dosage élémentaire quantitatif de l'élément zinc après minéralisation sulfonitrique par spectrométrie atomique, de façon à en déduire la quantité de sulfure de zinc. Il est également possible de déterminer quantitativement l'élément soufre par microanalyse et/ou de procéder à une dissolution de la matrice polymérique dans un solvant, une filtration de l'additif et une analyse par diffraction X.

La proportion en poids de sulfure de zinc par rapport au poids total de la composition, destinée à former les fils, fibres et/ou filaments, peut être comprise entre 0,01 et 10 %, préférentiellement entre 0,1 et 7 %, encore plus préférentiellement entre 0,2 et 5 %, particulièrement entre 0,3 et 3 %. La quantité de sulfure de zinc dans les fils, fibres et/ou filaments peut varier selon différents critères, tels que la matité, la formulation, le type de polymère, le mode d'introduction, le mode d'application, la nature des organismes nuisibles et l'environnement.

A titre d'exemple de polymères composant la matrice polymérique, on peut citer : les polylactones telles que la poly(pivalolactone), la poly(caprolactone) et les polymères de la même famille; les polyuréthanes obtenus par réaction entre des diisocyanates comme le 1,5-naphtalène diisocyanate; le p-phénylène diisocyanate, le m-phénylène diisocyanate, le 2,4-toluène diisocyanate, le 4,4'-diphénylméthane diisocyanate, le 3,3'-diméthyl-4,4'-diphényl-méthane diisocyanate, le 3,3-'diméthyl-4,4'-biphényl diisocyanate, le 4,4'-diphénylisopropylidène diisocyanate, le 3,3'-diméthyl-4,4'-diphényl diisocyanate, le 3,3'-diméthyl-4,4'-diphénylméthane diisocyanate, le 3,3'-diméthoxy-4,4'-biphényl diisocyanate, le dianisidine diisocyanate, le toluidine diisocyanate, le hexaméthylène diisocyanate, le 4,4'-düsocyanatodiphénylméthane et composés de la même famille et les diols à longues chaînes linéaires comme le poly(tétraméthylène adipate), le poly(éthylène adipate), le poly(1,4 -butylène adipate), le poly(éthylène succinate), le poly(2,3-butylène succinate), les polyéther diols et composés de la même famille; les polycarbonates comme le poly[méthane bis(4-phényl) carbonate], le poly[1,1-éther bis(4-phényl) carbonate], le poly[diphénylméthane bis(4-phényl)carbonate], le poly[1,1-cyclohexane bis(4-phényl)carbonate] et polymères de la même famille; les polysulfones; les polyéthers; les polycétones; les polyamides comme le poly(4-amino butyrique acide), le poly(héxaméthylène adipamide), le poly(-ca p rolactame) le poly(acide 6-aminohéxanoïque), le poly(m-xylylène adipamide), le poly(p-xylylène sébacamide), le poly(2,2,2-triméthyl héxaméthylène téréphtalamide), le poly(métaphénylène isophtalamide), le poly(p-phénylène téréphtalamide), et polymères de la même famille; les polyesters comme le poly(éthylène azélate), le poly(éthylène-1,5-naphtalate, le poly(1,4-cyclohexane diméthylène téréphtalate), le poly(éthylène oxybenzoate), le poly(para-hydroxy benzoate), le poly(1,4-cyclohéxylidène diméthylène téréphtalate), le poly(1,4-cyclohéxylidène diméthylène téréphtalate), le polyéthylène téréphtalate, le polybutylène téréphtalate et les polymères de la même famille; les poly(arylène oxydes) comme le poly(2,6-diméthyl-1,4-phénylène oxyde), le poly(2,6-diphényl-1,4-phénylène oxyde) et les polymères de la même famille ; les poly(arylène sulfides) comme le poly(phénylène sulfide) et les polymères de la même famille; les polyétherimides; les polymères vinyliques et leurs copolymères comme l'acétate de polyvinyle, l'alcool polyvinylique, le chlorure de polyvinyle; le polyvinyle butyral, le chlorure de polyvinylidène, les copolymères éthylène- acétate de vinyle, et les polymères de la même famille; les polymères acryliques, les polyacrylates et leurs copolymèrés comme l'acrylate de polyéthyle, le poly(n-butyl acrylate), le polyméthylméthacrylate, le polyéthyl méthacrylate, le poly(n-butyl méthacrylate), le poly(n-propyl méthacrylate), le polyacrylamide, le polyacrylonitrile, le poly(acide acrylique), les copolymères éthylène- acide acrylique, les copolymères éthylène-alcool vinylique, les copolymères de l'acrylonitrile, les copolymères méthacrylate de méthyle -styrène , les copolymères éthylène-acrylate d'éthyle, les copolymères méthacrylate-butadiène-styrène, l'ABS, et les polymères de la même famille; les polyoléfines comme le poly(éthylène) basse densité, le poly(propylène), le poly(éthylène) chloré basse densité, le poly(4-méthyl-1-pentène), le poly(éthylène), le poly(styrène), et les polymères de la même famille; les ionomères; les poly(épichlorohydrines); les poly(uréthane) tels que produits de polymérisation de diols comme la glycérine, le triméthylol-propane, le 1,2,6-hexanetriol, le sorbitol, le pentaérythritol, les polyéther polyols, les polyester polyols et composés de la même famille avec des polyisocyanates comme le 2,4-tolylène diisocyanate, le 2,6-tolylène diisocyanate, le 4,4'-diphénylméthane diisocyanate, le 1,6-héxaméthylène diisocyanate, le 4,4'-dicycohéxylméthane diisocyanate et les composés de la même famille; et les polysulfones telles que les produits de réaction entre un sel de sodium du 2,2-bis(4-hydroxyphényl) propane et de la 4,4'-dichlorodiphényl sulfone; les résines furane comme le poly(furane); les plastiques cellulose-ester comme l'acétate de cellulose, l'acétate-butyrate de cellulose, propionate de cellulose et les polymères de la même famille; les silicones comme le poly(diméthyl siloxane), le poly(diméthyl siloxane co-phénylméthyl siloxane), et les polymères de la même famille; les mélanges d'au moins deux des polymères précédents.

Comme autre matrice polymérique, on peut citer également par exemple la viscose, la cellulose et l'acétate de cellulose ; les polyamide imides ou les polyimides; les latex tels que les latex acryliques et uréthane.

La matrice polymérique de l'invention peut également être du type des polymères utilisés dans les adhésifs, tels que par exemple les copolymères d'acétates de vinyles plastisol, les latex acryliques, les latex uréthanes et les PVC plastisol.

La matrice polymérique est préférentiellement une matrice thermoplastique.

Préférentiellement, les fils, fibres et/ou filaments de la présente invention comprennent une matrice thermoplastique composé d'un polymère thermoplastique choisi dans le groupe comprenant les polyamides; les polyesters tels que le polyéthylène téréphtalate (PET), le polybutylène téréphtalate (PBT), le polytriméthylène téréphtalate (PTT); les polyoléfines tels que le polypropylène, le polyéthylène; le chlorure de polyvinylidène (PVC), leurs copolymères et mélanges.

Préférentiellement, la matrice thermoplastique comprend au moins un polyamide choisi dans le groupe comprenant : le polyamide 6, le polyamide 6.6, le polyamide 11, le polyamide 12, le polyamide 4, les polyamides 4-6, 6-10, 6-12, 6-36, 12-12, leurs copolymères et mélanges, tel qu'un mélange de polyamide 6 et 6.6. On peut également utiliser différents types de polyamides aromatiques.

Selon une variante particulière de l'invention, la matrice thermoplastique est un polymère comprenant des chaînes macromoléculaires étoiles ou H, et le cas échéant des chaînes macromoléculaires linéaires. Les polymères comprenant de telles chaînes macromoléculaires étoiles ou H sont par exemple décrits dans les documents FR 2743077, FR 2779730, US 5959069, EP 0632703, EP 0682057 et EP 0832149.

La matrice thermoplastique de l'invention peut également être un polymère de type arbre statistique, de préférence un copolyamide présentant une structure arbre statistique. Ces copolyamides de structure arbre statistique ainsi que leur procédé d'obtention sont notamment décrits dans le document WO 99/03909. La matrice thermoplastique de l'invention peut également être une composition comprenant un polymère thermoplastique linéaire et un polymère thermoplastique étoile, H et/ou arbre tels que décrits ci-dessus. La matrice thermoplastique de l'invention peut également comprendre un copolyamide hyperbranché du type de ceux décrits dans le document WO 00/68298. La matrice thermoplastique de l'invention peut également comprendre toute combinaison de polymère thermoplastique étoile, H, arbre, copolyamide hyperbranché décrit ci-dessus.

Le sulfure de zinc peut se présenter sous la forme de particules. Les particules de sulfure de zinc peuvent présenter un diamètre inférieur ou égal à 5 µm, préférentiellement inférieur ou égal à 1 µm, plus préférentiellement compris entre 0,1 et 0,5 µm, particulièrement un diamètre d'environ 0,3 µm.

Préférentiellement, les fils, fibres et/ou filaments de la présente invention comprennent exclusivement du sulfure de zinc en tant qu'agent antibactérien et antifongique. Toutefois, le sulfure de zinc peut être utilisé en association avec au moins un autre agent anti-microbien tels que par exemple l'argent, l'oxyde d'argent, un halogénure d'argent, l'oxyde de cuivre (I), l'oxyde de cuivre (II), le sulfure de cuivre, l'oxyde de zinc et le silicate de zinc, l'homme du métier étant à même de choisir la nature et la proportion d'agent antimicrobien selon l'utilisation, le mode d'application, la nature des organismes nuisibles, la nature des fibres, fils, filaments et/ou articles et l'environnement.

Le sulfure de zinc introduit dans la matrice polymérique peut être sous forme de particules qui sont ni enrobées ni encapsulées. Toutefois, ces particules peuvent également être enrobées et/ou encapsulées. Les particules de sulfure de zinc peuvent être enrobées et/ou encapsulées par au moins un composé minéral et/ou organique. On peut utiliser des particules de sulfure de zinc ne comportant pas d'enrobage minéral.

Les fils, fibres, filaments et/ou articles de la présente invention peuvent aussi contenir tous les autres additifs pouvant être utilisés, par exemple des charges de renfort, des ignifugeants, des stabilisants aux UV, à la chaleur, des pigments, et des matifiants tels que le dioxyde de titane.

La présente invention concerne également un procédé de fabrication de fils, fibres et/ou filaments à propriétés antibactérienne et antifongique consistant à filer une composition comprenant une matrice polymérique, préférentiellement thermoplastique, et du sulfure de zinc.

Le mélange du sulfure de zinc et de la matrice polymérique peut être effectué de différentes manières bien connues de l'homme du métier. Les compositions comprenant une matrice polymérique et du sulfure de zinc selon l'invention sont de préférence réalisées par introduction du sulfure de zinc dans le polymère fondu dans un dispositif de mélange, par exemple en amont d'un dispositif de filage. Elles peuvent également être réalisées par introduction du sulfure de zinc dans une solution de polymère, par exemple en amont d'un dispositif de filage par voie humide. Les compositions peuvent également être réalisées par introduction du sulfure de zinc avant la polymérisation (avec les matières premières) et/ou au cours de la polymérisation de la matrice polymérique, préférentiellement thermoplastique. On peut ajouter à la matrice polymérique une composition concentrée (masterbatch) à base de matrice polymérique comprenant du sulfure de zinc.

On peut notamment utiliser le procédé suivant comprenant au moins les étapes :
- a) mise en contact de la matrice polymérique, éventuellement à l'état fondu, avec du sulfure de zinc et/ou une composition concentrée à base de matrice polymérique comprenant du sulfure de zinc ; et
- b) filage du mélange obtenu à l'étape a) de façon à obtenir des fils, fibres et/ou filaments.

Les compositions peuvent être mises en forme de fils fibres et/ou filaments, directement après la polymérisation, sans étapes intermédiaires de solidification et de refonte. Elles peuvent aussi êtres mises en forme de granulés, destinés à subir une refusion pour mise en forme définitive ultérieure, par exemple pour la fabrication d'articles moulés ou pour la fabrication de fils fibre ou filaments.

Tous les procédés de filage en fondu peuvent être utilisés.

Pour la fabrication de fils multifilamentaires, on cite les procédés de filage ou filage-étirage ou filage-étirage-texturation intégrés ou non, quelle que soit la vitesse de filage. On peut produire les fils par filage haute vitesse, à vitesse de filage supérieure à 3500 m/min. De tels procédés sont souvent désignés par les termes suivants: POY (partialy oriented yarn), FOY (fully oriented yarn), FEI (filage-étirage-intégré).

Pour la fabrication de fibres, les filaments peuvent par exemple être réunis sous forme de mèche ou de nappe, directement après le filage ou en reprise, étirés, texturés ou frisés et coupés. Les fibres obtenues peuvent être utilisées pour la fabrication de non tissés ou de filés de fibres. Les compositions peuvent également être utilisées pour la fabrication de flock.

Il est également possible de produire des fils, fibres et/ou filaments bicomposés dont certaines parties comprennent du sulfure de zinc.

Les fils, fibres et/ou filaments de l'invention peuvent subir divers traitements, tels que par exemple l'étirage en une étape continue ou en reprise, le dépôt d'ensimage, l'huilage, l'entrelacement, la texturation, le frisage, l'étirage, le traitement thermique de fixation ou de relaxation, le moulinage, le retordage, et/ou la teinture. Pour la teinture, on cite en particulier les procédés de teinture en bain ou par jets. Les teintures préférées sont les teintures acides, métallifères ou non métallifères.

La présente invention concerne aussi un article à propriétés antibactérienne et antifongique obtenu au moins à partir de fils, fibres et/ou filaments tel que définis précédemment. Ces articles peuvent être des étoffes ou des surfaces textiles, telles que des surfaces tissées, tricotées, non-tissées ou tapis. En effet, les fils, fibres, filaments, articles et/ou articles composites peuvent être mis en oeuvre dans la fabrication de tout article susceptible d'être en contact avec des bactéries et/ou champignons, tels que par exemple les moquettes, les tapis, les revêtements d'ameublement, les revêtements de surface, les canapés, les rideaux, la literie, les matelas et oreillers, les vêtements et les matériaux textiles médicaux.

De tels articles peuvent être obtenus notamment à partir d'un seul type de fils, fibres, et/ou filaments ; ou au contraire à partir d'un mélange de fils, fibres, et/ou filaments de types différents. L'article comprend au moins en partie des fils, fibres et/ou filaments selon l'invention. Pour un type donné de fils, fibres, filaments, par exemple des fils, fibres, filaments ne contenant pas de sulfure de zinc, des fils, fibres ou filaments de natures différentes peuvent être utilisés dans l'article de l'invention. La présente invention concerne également des articles composites à propriétés antibactérienne et antifongique comprenant au moins un article selon l'invention. Les articles composites sont des articles à plusieurs composants. Ces composants peuvent être par exemple des fibres courtes, des supports, des articles obtenus à partir de fils, fibres, filaments tels que des articles non tissés. Dans le cadre de l'invention, au moins un des composants de l'article textile composite comprend du sulfure de zinc.

La présente invention concerne aussi des articles obtenus par mise en forme d'une composition à base de matrice polymérique comprenant au moins du sulfure de zinc. Ces articles peuvent notamment être obtenus par un procédé choisi dans le groupe comprenant un procédé d'extrusion, tel que l'extrusion de feuilles et de films, de moulage, tel que le moulage par compression, et d'injection, tel que le moulage par injection. Des films peuvent ainsi être obtenus par les procédés mentionnés précédemment en utilisant une filière plate. Préférentiellement, la matrice thermoplastique est composée de polyamide, de polyester ou de polyoléfine. Les films obtenus peuvent subir une ou différentes étapes de traitements, telles qu'un étirage unidimensionnelle ou bidimensionnelle, un traitement thermique de stabilisation, un traitement antistatique ou un ensimage.

### Exemple 1 : Préparation des échantillons

Un polyamide 66 standard ayant une viscosité relative de 2,6 (mesuré à 1 g/100 mL dans l'acide sulfurique à 96 % à 25°C) est séché d'une manière conventionnelle pour obtenir une humidité résiduelle de 0,09%. Il est ensuite réduit en poudre et mélangé avec 2% de poudre de ZnS (Sachtolith HD-S de Sachtleben - Germany). Le mélange résultant est fondue dans une extrudeuse et filé dans une filière possédant 10 trous de filière, créant ainsi 10 filaments qui sont refroidis par soufflage d'air (20°C, humidité relative 66%). Les filaments sont alors réunis et huilés avec une émulsion standard à 8,6%, puis renvidés sur un tube à 4200 m/min. Le fils résultant partiellement orienté (POY), ayant un titre global de 42 dtex, est alors tricoté par une machine conventionnelle pour obtenir un article (une chaussette). Cet article est alors sujet à un cycle de teinture dans les conditions suivantes :
- Désensimage à 60°C pendant 20 min avec 1 g/L d'un détergent anionique (Invatex CRA de CIBA) et 1g/L de carbonate de sodium.
- Teinture en bain ouvert (volume 7 L), en chauffant de 1,6°C/min, puis en maintenant 45 min à 98°C. La recette contient 1% de Nylosan Bleu NBLN (Clariant), 1% de Sandogen NH (égalisateur de Clariant), 1 g/L de Sandacid VA (donneur d'acide de Clariant) et 0,5 g/L d'acétate de sodium.

Un article obtenu sans addition de ZnS a également été fabriqué dans les mêmes conditions en tant qu'échantillon témoin pour les tests antibactériens et antifongiques.

### Exemple 2 : Test antibactérien

L'activité antibactérienne est mesurée selon la norme JIS L 1902: 1998, en suivant le mode opératoire particulier du Laboratoire d'Hygiene et de Biotechnologie du Hohenstein Institut (Allemagne) :
- on utilise les bactéries *Staphylococcus aureus* ATCC 6538P et Klebsiella pneumoniae DSM 789, initialement maintenue à l'état sec et congelé. Les cultures sont inoculées sur une base nutritive (LAB8, LabM), et incubées à 37°C pendant 48 heures. Les bactéries sont ensuite transférées dans des erlenmeyers de 250 ml, sur une base nutritive (LAB14, LabM) et incubées à 37°C pendant 18 heures. On dilue la culture à 1/200 avec une solution saline isotonique (NaCl 0,85% poids + 0,05% Tween 80), de façon à ce que la suspension comprenne environ 10⁵ bactéries par ml.
- Les tests sont effectués sur des surfaces tricotées de 18 mm sur 18 mm. On utilise autant de surfaces que nécessaires pour absorber exactement 0,2 ml de suspension.

Les échantillons testés sont un échantillon témoin et un échantillon selon l'invention.

Les surfaces tricotées sont placées dans des bouteilles de 30 ml. On prépare six bouteilles comprenant des échantillons témoin et trois bouteilles pour l'échantillon selon l'invention à tester. Les bouteilles sont couvertes d'un film, et stérilisées à 121°C pendant 15 minutes.

On inocule les bactéries aux échantillons compris dans les bouteilles avec les 0,2 ml de la suspension de bactéries, en prenant soin de ne pas mettre en contact la suspension avec les parois de la bouteille. Immédiatement après l'inoculation, on ajoute 20 ml d'une solution isotonique Tween 80 (0,2% en poids) à trois des bouteilles contenant l'échantillon témoin, on les ferme à l'aide d'un bouchon stérile, et on les agite pendant 30 secondes. On dénombre ensuite le nombre de bactéries.

On place les autres bouteilles dans un dessiccateur, et on laisse incuber les bactéries pendant 18 heurs à 37°C. Après incubation le nombre de bactéries est compté, de la même manière que le nombre de bactéries au temps zéro.

On détermine en particulier les quantités suivantes :
A = nombre moyen de bactéries actives immédiatement après l'inoculation sur l'échantillon témoin
B = nombre moyen de bactéries actives après 18 heures d'incubation sur l'échantillon témoin
C = nombre moyen de bactéries actives après 18 heures d'incubation sur l'échantillon selon l'invention (avec ZnS)
F = facteur de croissance = Log(B) - Log(A). Le test est jugé valide si F > 0 ±0,5
S = activité spécifique = Log(B) - Log(C)
Cfu (colony forming unit) : unité formant une colonie

Les résultats sont résumés dans les tableaux 1 et 2, pour les bactéries Gram + et Gram -.

**Tableau 1**

| *Staphylococcus aureus* (Gram +) : Souche ATCC 6538P | | | | | | |
|---|---|---|---|---|---|---|
| | **Echantillon** | **Echantillon** | **Echantillon** | **Moyenne** | **Moyenne** | |
| | **1 (cfu)** | **2 (cfu)** | **3 (cfu)** | **(cfu)** | **(Log cfu)** | |
| Témoin 0 h | 4,50 x 10⁵ | 3,60 x 10⁵ | 4,50 x 10⁵ | 4,20 x 10⁵ | 5,62 | |
| Témoin 18 h | 4,64 x 10⁵ | 8,39 x 10⁵ | 8,70 x 10⁵ | 7,25 x 10⁵ | 5,86 | F = 0,24 |
| Test 18 h | < 20 | 4,07 x 10² | < 20 | 1,36 x 10² | 2,13 | S = 3,87 |

**Tableau 2**

| *Klebsiella pneumoniae* (Gram -) : Souche DSM 789 | | | | | | |
|---|---|---|---|---|---|---|
| | **Echantillon** | **Echantillon** | **Echantillon** | **Moyenne** | **Moyenne** | |
| | **1 (cfu)** | **2 (cfu)** | **3 (cfu)** | **(cfu)** | **(Log cfu)** | |
| Témoin 0 h | 2,15 x 10⁵ | 6,70 x 10⁵ | 7,40 x 10⁵ | 5,42 x 10⁵ | 5,73 | |
| Témoin 18 h | 3,58 x 10⁷ | 4,00 x 10⁷ | 4,28 x 10⁷ | 3,95 x 10⁷ | 7,60 | F = 1,86 |
| Test 18 h | 2,28 x 10⁷ | 2,71 x 10⁷ | 2,76 x 10⁷ | 2,58 x 10⁷ | 7,41 | S = 0,18 |

Ainsi, il apparaît que les articles obtenus à partir de fils comprenant du ZnS présentent une forte activité antibactérienne sur les bactéries Gram+ et Gram-.

### Exemple 3 : Permanence de l'activité antibactérienne après lavages

Les deux échantillons (témoin et selon l'invention) préparés précédemment sont lavés 30 fois selon la norme EN 26330 - protocole 5A : la température de lavage est 40°C, le détergent utilisé est sans « blanchisseur optique » et la machine utilisée est une machine domestique standard. Les échantillons sont séchés à température ambiante.

L'activité antibactérienne est ensuite de nouveau mesurée selon la même méthodologie que précédemment. Les résultats sont rassemblés dans les tableaux 3 et 4.

**Tableau 3**

| *Staphylococcus aureus* (Gram +) : Souche ATCC 6538P | | | | | | |
|---|---|---|---|---|---|---|
| | **Echantillon** | **Echantillon** | **Echantillon** | **Moyenne** | **Moyenne** | |
| | **1 (cfu)** | **2 (cfu)** | **3 (cfu)** | **(cfu)** | **(Log cfu)** | |
| Témoin 0 h | 3,40 x 10⁵ | 3,10 x 10⁵ | 3,80 x 10⁵ | 3,43 x 10⁵ | 5,54 | |
| Témoin 18 h | 8,10 x 10² | <20 | <20 | 2,71 x 10² | 2,43 | F = - 3,1 |
| Test 18 h | <20 | <20 | <20 | <20 | 0,01 | S = 2,42 |

**Tableau 4**

| *Klebsiella pneumoniae* (Gram -) : Souche DSM 789 | | | | | | |
|---|---|---|---|---|---|---|
| | **Echantillon** | **Echantillon** | **Echantillon** | **Moyenne** | **Moyenne** | |
| | **1 (cfu)** | **2 (cfu)** | **3 (cfu)** | **(cfu)** | **(Log cfu)** | |
| Témoin 0 h | - | - | - | - | - | |
| Témoin 18 h | 3,00 x 10⁶ | 2,70 x 10⁷ | 2,30 x 10⁷ | 1,77 x 10⁷ | 7,25 | - |
| Test 18 h | 1,10 x 10⁶ | 1,10 x 10⁶ | 2,30 x 10⁶ | 1,50 x 10⁶ | 6,18 | S = 1,07 |

Ainsi, il apparaît que les articles obtenus à partir de fils comprenant du ZnS présentent une forte activité antibactérienne sur les bactéries Gram+ et Gram-, même après 30 lavages.

### Exemple 4 : Test antifongique

L'évaluation de l'activité antifongique (antimycosique) est mesurée selon la norme ASTM E 2149-01 (shake flask test), en suivant le mode opératoire adapté par le Laboratoire d'Hygiene et de Biotechnologie du Hohenstein Institut (Allemagne) pour les champignons. 1 g de produit à tester est mis en contact avec 70 ml d'une solution de sels minéraux et 5 ml d'une suspension d'*Aspergillus niger à* 1-3.10⁵ CFU/ml dans un erlenmeyer de 250 ml. La solution de sels minéraux a été préalablement préparée avec la composition exacte suivante :

| | |
|---|---|
| NaNO₃ | 2,0 g |
| KH₂PO₄ | 0,7 g |
| K₂HPO₄ | 0,3 g |
| KCI | 0,5 g |
| MgSO₄ x 7 H₂O | 0,5 g |
| FeSO₄ x 7 H₂O | 0,01 g |
| H₂O | 1000 ml |
| TWEEN 80 | 0,1 g |

Un erlenmeyer est réalisé de manière similaire avec 1 g de l'échantillon témoin.

Les erlenmeyers sont agités à 300 mouvements par minute à température ambiante. Un dénombrement des champignons est effectué après 0 et 3 jours d'incubation.

On définit un taux de réduction R de la manière suivante :
R=100x(B-A)/B
A = cfu par millilitre pour l'erlenmeyer contenant l'échantillon après 3 jours de contact.
B = cfu par millilitre pour l'erlenmeyer avant le contact avec l'échantillon (temps 0)

Les résultats sont mentionnés dans le tableau 5 :

**Taleau 5**

| *Aspergillus niger* "von Thieghem" : Souche ATCC 6275 (DSM 1957) | | | |
|---|---|---|---|
| | **Temps 0** | **Temps 3** | **R** |
| | **(cfu/ml)** | **jours (cfu/ml)** | |
| Témoin | > 1,00 x 10⁶ | 1,90 x 10⁶ | R = -90% |
| | | | (augmentation) |
| Test | 8,00 x 10⁵ | 1,00 x 10⁴ | R = 99 % (réduction) |

Ainsi, il apparaît que les articles obtenus à partir de fils comprenant du ZnS présentent une forte activité antifongique.

### Exemple 5 : Préparation de bobines de fils et caractérisation

L'indice de jaune et la dégradation de la matrice polyamide ont été comparés sur des fils comprenant du ZnS et des fils comprenant du ZnO.

Le polyamide 66 (PA66) mis en oeuvre est un polyamide ne comprenant pas de dioxyde de titane, de viscosité relative de 2,5 (mesurée à une concentration de 1 g/100mL dans de l'acide sulfurique à 96%, à 25°C).

L'incorporation du ZnS ou du ZnO dans le PA66 se fait par mélange. Le mélange est séché 20h à 100°C sous vide de 50 mbars environ puis introduit dans un dispositif d'extrusion double vis qui assure le mélange en phase fondue. Le taux d'incorporation de ZnS ou du ZnO, mentionné dans le tableau suivant, est calculé par rapport au poids total de la composition. Il est ensuite procédé au filage du mélange fondu avec une température en tête de filière adéquate permettant l'obtention d'un fil (les températures de filages sont mentionnées dans le tableau suivant) et une vitesse au premier point d'appel de 4200 m/min, de manière à obtenir un fil continu multifilamentaire de 42 dtex pour 10 filaments. Le multifilament ou fil est constitué de 10 brins (la filière est constituée de 10 trous de 0.38mm) et le diamètre d'un brin est d'environ 20 µm.

Les fils obtenus ont été caractérisés par une mesure de la masse moléculaire de la matrice polyamide par GPC (chromatographie par perméation de gel) dans le dichlorométhane après dérivatisation avec l'anhydride trifluoroacétique, par rapport à des solutions étalons de polystyrène (PS). La technique de détection utilisée est la réfractométrie. La masse moléculaire de la matrice est estimée comme le maximum du pic réfractométrique.

Les fils ont été également caractérisés par une mesure d'indice de jaune selon la norme YI DIN 6167 (source illuminant D65).

Les résultats sont reportés sur le tableau 6 :

**Tableau 6**

| **Composition** | **Température de** | **Indice de Jaune** | **GPC** |
|---|---|---|---|
| | **filage (°C)** | | **(g/mol equiv. PS)** |
| PA 66 témoin | 283 | 8,7 | 65 000 |
| PA 66 + 0,24% ZnS | 283 | 9,4 | 65 000 |
| PA 66 + 0,5% ZnS | 283 | 9,2 | 67 000 |
| PA 66 + 0,2% ZnO | 280 | 13,5 | 56 000 |
| PA 66 + 0,5% ZnO | 271 | 14,8 | 52 000 |

Ainsi, le ZnS présente dans des fils des capacités bien plus intéressantes que le ZnO en matière de résistance au jaunissement et de préservation de la matrice polyamide. Le ZnS est par conséquent plus apte à être introduit dans des matrices, pour l'obtention de fils, par rapport au ZnO connu pour ses propriétés antimicrobiennes.

### Exemple 6 : Test antifongique en comparaison avec poudre ZnS

Le champignon utilisé est l'*Eurotium amstelodami* (souche CBS 11248). Il est cultivé dans un milieu à 20g/l d'extrait de malt, 200 g/l de sucrose et 15 g/l d'agar.

Les échantillons testés comprennent les produits de base suivants :
- Une poudre de polyamide 6 de viscosité relative 2,6 (mesurée à 1g/100mLdans l'acide sulfurique à 96 %, à 25°C), broyée à une granulométrie inférieure à 500µm ;
- Un masterbatch à 40% en poids de ZnS dans du polyamide 6 (référence Sachtolen PA ZS 40 de Sachtleben, comprenant du Sachtolith HD-S de Sachtleben) broyé à une granulométrie inférieure à 500 µm ; et
- Une poudre de ZnS (Sachtolith HD-S de Sachtleben).

4 milieux de culture différents ont été fabriqués :
- milieu 1 : 20g/l d'extrait de malt, 200 g/l de sucrose et 15 g/l d'agar ;
- milieu 2 : milieu 1 contenant 7,5% en poids de poudre de PA 6 ;
- milieu 3 : milieu 1 contenant 12,5% en poids de poudre de masterbatch à 40% ; soit 5% en équivalent ZnS et 7,5% en équivalent PA 6 ; et
- milieu 4 : milieu 1 contenant un mélange de poudre : 5% en poids de poudre de ZnS et 7,5% en poids de poudre de PA 6 (le polyamide ne comprenant pas du ZnS).

Ces quatre milieux ont été stérilisés avant d'être versés dans des boites de Pétri de 85 mm de diamètre.

Des spores de *E. amstellodami* ont été collectés sur une culture de 3 semaines, placées en suspension dans un milieu contenant 1/1000 de peptone et 1/1000 de Tween 80 puis diluées jusqu'à obtenir 10⁶ spores/ml.

30 µl de suspension a été déposé au centre de chaque milieu à tester. 3 répliques ont été faites pour chaque milieu.

Les boites de pétri ont ensuite été incubées à 25°C sous lumière blanche constante.

A 12 et 16 jours d'incubation, le diamètre de la colonie a été mesuré sur chacun des milieux. Les résultats des moyennes des trois répliques sont portés dans le tableau suivant :

**Tableau 7**

| | **Diamètre de la colonie (mm)** | | | |
|---|---|---|---|---|
| **Nombre de jours d'incubation** | **Milieu 1** | **Milieu 2** | **Milieu 3** | **Milieu 4** |
| 0 | 1 | 1 | 1 | 1 |
| 12 | 78 | 80 | 55 | 61 |
| 16 | 85 | 85 | 60 | 69 |

La variabilité est de plus ou moins 1 mm entre les différentes répliques.

Ainsi, il apparaît que le ZnS contenu dans le PA6 cause une forte diminution de la croissance du champignon.

### Exemple 7 : Test antibactérien en comparaison avec poudre ZnS

Les échantillons testés comprennent les produits de base suivants :
- Une poudre de polyamide 6 (ci-après appelé poudre A) de viscosité relative 2,6 (mesurée à 1g/100mLdans l'acide sulfurique à 96 %, à 25°C) ; et
- Une poudre de ZnS (Sachtolith HD-S de Sachtleben).

L'activité antibactérienne est mesurée selon la même méthodologie que dans l'exemple 2, hormis le fait que l'on met en contact de la poudre avec la suspension bactérienne.
- Témoin : (A) extr :
   Poudre obtenue par extrusion de la poudre A. L'extrusion de déroule comme suit : la poudre est séchée 16 heures à 80°C sous vide de 50 mbars environ, puis introduit dans un dispositif d'extrusion double vis. Les caractéristiques de marche de l'extrudeuse double vis sont les suivantes : Température du fondu : 240°C environ ; Temps de séjour en fondu : 3 minutes. Le jonc obtenu en sortie du dispositif d'extrusion est trempé dans de l'eau à 20°C environ puis concassé et broyé après refroidissement avec de la carboglace avec un broyeur ultra centrifuge Retsch ZM 1000. La granulométrie de la poudre obtenue est inférieure à 500 µm.
- Test 1 : (A+ZnS)extr. :
   Poudre obtenue par mélange de 2 % en poids de ZnS sous forme de poudre avec de la poudre A et extrusion du mélange de poudre, comme mentionné ci-dessus. Ainsi, la poudre obtenue comprend des granulés de polyamide 6 comprenant du ZnS.
- Test 2 : (A)extr + ZnS

Poudre obtenue par mélange de 2 % en poids de ZnS sous forme de poudre avec la poudre du témoin (A) extr. Ainsi, la poudre obtenue comprend des granulés de polyamide 6 et du ZnS.

Les résultats sont rassemblés dans le tableau suivant.

**Tableau 8**

| *Staphylococcus aureus* (Gram +) : Souche ATCC 6538P | | | | | | |
|---|---|---|---|---|---|---|
| | **Echantillon** | **Echantillon** | **Echantillon** | **Moyenne** | **Moyenne** | |
| | **1 (cfu)** | **2 (cfu)** | **3 (cfu)** | **(cfu)** | **(Log cfu)** | |
| Témoin 0 h | 1,20 x 10⁵ | 1,20 x 10⁵ | 1,50 x 10⁵ | 1,3 x 10⁵ | 5,11 | |
| Témoin 18 h | 2,90 x 10⁵ | 2,70 x 10⁵ | 3,00 x 10⁵ | 2,87 x 10⁵ | 5.46 | |
| Test 1 18 h | 1,50 x 10⁴ | 2,50 x 10⁴ | 3,70 x 10⁴ | 2,57 x 10⁴ | 4,41 | S = 1,05 |
| Test 2 18 h | 3,10 x 10⁵ | 6,30 x 10⁵ | 5,60 x 10⁵ | 5,00 x 10⁵ | 5,70 | S = - 0,24 |

Ce test montre de façon surprenante que l'activité antibactérienne du ZnS est obtenue lorsque le ZnS est mélangé au sein de la matrice polymérique.

## Revendications

1. Utilisation de sulfure de zinc dans une matrice polymérique pour la fabrication de fils, fibres, filaments ou articles à propriétés antibactérienne et antifongique.

2. Utilisation selon la revendication 1, **caractérisée en ce que** la proportion en poids de sulfure de zinc par rapport au poids total de la composition destinée à former des fils, fibres ou filaments est comprise entre 0,01 et 10 %.

3. Utilisation selon la revendication 1 ou 2, **caractérisée en ce que** la proportion en poids de sulfure de zinc par rapport au poids total de la composition destinée à former des fils, fibres ou filaments est comprise entre 0,2 et 5 %.

4. Utilisation selon l'une quelconque des revendications 1 à 3, **caractérisée en ce que** la matrice polymérique est une matrice thermoplastique.

5. Utilisation selon l'une quelconque des revendications 1 à 4, **caractérisée en ce que** la matrice thermoplastique comprend au moins un polymère thermoplastique choisi dans le groupe comprenant les polyamides, les polyesters tels que le PET, le PBT, le PTT; les polyoléfines tels que le polypropylène, le polyéthylène; le PVC; leurs copolymères et mélanges.

6. Utilisation selon l'une quelconque des revendications 1 à 5, **caractérisée en ce que** la matrice thermoplastique comprend au moins un polyamide choisi dans le groupe comprenant: le polyamide 6, le polyamide 6.6, le polyamide 11, le polyamide 12, le polyamide 4, les polyamides 4-6, 6-10, 6-12, 6-36, 12-12; leurs copolymères et mélanges.

7. Utilisation selon l'une quelconque des revendications 1 à 6, **caractérisée en ce que** le sulfure de zinc est sous forme de particules enrobées et/ou encapsulées par au moins un composé minéral et/ou organique.

8. Utilisation selon l'une des revendications 1 ou 4 à 7, **caractérisée en ce que** l'article est un article composite comprenant au moins des fils, fibres ou filaments tels que décrits à l'une des revendications 1 à 7.

9. Utilisation selon l'une des revendications 1 à 7, **caractérisée en ce que** les fils, fibres ou filaments sont fabriqués par filage d'une composition comprenant une matrice polymérique et du sulfure de zinc.

10. Utilisation selon la revendication 9, **caractérisée en ce que** le procédé de fabrication comprend au moins les étapes suivantes :
- a) mise en contact de la matrice polymérique, éventuellement à l'état fondu, avec du sulfure de zinc et/ou une composition concentrée à base de matrice polymérique comprenant du sulfure de zinc ; et
- b) filage du mélange obtenu à l'étape a) de façon à obtenir des fils, fibres et/ou filaments.

## Claims

1. Use of zinc sulfide in a polymer matrix for the manufacture of yarns, fibres, filaments or articles with antibacterial and antifungal properties.

2. Use according to Claim 1, **characterized in that** the weight proportion of zinc sulfide relative to the total weight of the composition intended to form yarns, fibres or filaments is between 0.01% and 10%.

3. Use according to Claim 1 or 2, **characterized in that** the weight proportion of zinc sulfide relative to the total weight of the composition intended to form yarns, fibres or filaments is between 0.2% and 5%.

4. Use according to any one of Claims 1 to 3, **characterized in that** the polymer matrix is a thermoplastic matrix.

5. Use according to any one of Claims 1 to 4, **characterized in that** the thermoplastic matrix comprises at least one thermoplastic polymer chosen from the group comprising polyamides, polyesters such as PET, PBT and PTT; polyolefins such as polypropylene and polyethylene; PVC; copolymers and blends thereof.

6. Use according to any one of Claims 1 to 5, **characterized in that** the thermoplastic matrix comprises at least one polyamide chosen from the group comprising: polyamide 6, polyamide 6,6, polyamide 11, polyamide 12, polyamide 4, polyamides 4-6, 6-10, 6-12, 6-36 and 12-12; copolymers and blends thereof.

7. Use according to any one of Claims 1 to 6, **characterized in that** the zinc sulfide is in the form of particles coated and/or encapsulated with at least one mineral and/or organic compound.

8. Use according to one of Claims 1 and 4 to 7, **characterized in that** the article is a composite article comprising at least yarns, fibres or filaments as described in one of Claims 1 to 7.

9. Use according to one of Claims 1 to 7, **characterized in that** the yarns, fibres or filaments are manufactured by spinning a composition comprising a polymer matrix and zinc sulfide.

10. Use according to Claim 9, **characterized in that** the manufacturing process comprises at least the following steps:
- a) placing the polymer matrix, optionally in melt form, in contact with zinc sulfide and/or a concentrated composition based on polymer matrix comprising zinc sulfide; and
- b) spinning the mixture obtained in step a) so as to obtain yarns, fibres and/or filaments.

## Patentansprüche

1. Verwendung von Zinksulfid in einer Polymermatrix zur Herstellung von Fäden, Fasern, Filamenten oder Gegenständen mit antibakteriellen und antimykotischen Eigenschaften.

2. Verwendung nach Anspruch 1, **dadurch gekennzeichnet, daß** der Gewichtsanteil von Zinksulfid, bezogen auf das Gesamtgewicht der Zusammensetzung zur Bildung der Fäden, Fasern oder Filamente zwischen 0,01 und 10% liegt.

3. Verwendung nach Anspruch 1 oder 2, **dadurch gekennzeichnet, daß** der Gewichtsanteil von Zinksulfid, bezogen auf das Gesamtgewicht der Zusammensetzung zur Bildung der Fäden, Fasern oder Filamente zwischen 0,2 und 5% liegt.

4. Verwendung nach einem der Ansprüche 1 bis 3, **dadurch gekennzeichnet, daß** es sich bei der Polymermatrix um eine thermoplastische Matrix handelt.

5. Verwendung nach einem der Ansprüche 1 bis 4, **dadurch gekennzeichnet, daß** die thermoplastische Matrix mindestens ein thermoplastisches Polymer aus der Gruppe bestehend aus Polyamiden, Polyestern wie PET, PBT und PTT; Polyolefinen wie Polypropylen und Polyethylen; PVC und Copolymeren und Mischungen davon umfaßt.

6. Verwendung nach einem der Ansprüche 1 bis 5, **dadurch gekennzeichnet, daß** die thermoplastische Matrix mindestens ein Polyamid aus der Gruppe bestehend aus Polyamid 6, Polyamid 6.6, Polyamid 11, Polyamid 12, Polyamid 4, den Polyamiden 4-6, 6-10, 6-12, 6-36, 12-12 und Copolymeren und Mischungen davon umfaßt.

7. Verwendung nach einem der Ansprüche 1 bis 6, **dadurch gekennzeichnet, daß** das Zinksulfid in Form von Teilchen, die mit mindestens einer anorganischen und/oder organischen Verbindung beschichtet und/oder verkapselt sind, vorliegt.

8. Verwendung nach einem der Ansprüche 1 oder 4 bis 7, **dadurch gekennzeichnet, daß** es sich bei dem Gegenstand um einen Verbundgegenstand, der mindestens Fäden, Fasern oder Filamente gemäß einem der Ansprüche 1 bis 7 umfaßt, handelt.

9. Verwendung nach einem der Ansprüche 1 bis 7, **dadurch gekennzeichnet, daß** die Fäden, Fasern oder Filamente durch Verspinnen einer Zusammensetzung, die eine Polymermatrix und Zinksulfid umfaßt, hergestellt werden.

10. Verwendung nach Anspruch 9, **dadurch gekennzeichnet, daß** das Herstellungsverfahren die folgenden Schritte umfaßt:
- a) Inberührungbringen der Polymermatrix, gegebenenfalls in geschmolzenem Zustand, mit Zinksulfid und/oder einer konzentrierten Zusammensetzung auf Basis von Zinksulfid umfassender Polymermatrix und
- b) Verspinnen der in Schritt a) erhaltenen Mischung zu Fäden, Fasern und/oder Filamenten.
